# EUROPÄISCHE PATENTANMELDUNG

(11) **EP 1 095 643 A2**
(43) Veröffentlichungstag der Anmeldung: **02.05.2001**
(21) Anmeldenummer: 00810975.3
(22) Anmeldetag: 20.10.2000
(51) Int. Cl.: A61F 11/14

(54) **Muschelgehörschützer mit Kopfbügel**

(30) Priorität: 28.10.1999 DE 29918929 U
(71) Anmelder: Artilux Herzig AG, 4410 Liestal (CH)
(72) Erfinder: Herzig, Hugo, 4402 Frenkendorf (CH)
(74) Vertreter: Heinen, Detlef

(57) **Zusammenfassung**

Ein Muschelgehörschützer wird vorgeschlagen mit einem elastischen Kopfbügel (**1**), mit beidseits am Kopfbügel (**1**) jeweils einer angeordneten Muschel (**6**), sowie mit zumindest einem Tragelement (**2**) am Kopfbügel (**1**) und mit zumindest einem komplementären Befestigungsorgan (**7**) an jeder Muschel (**6**). Das Befestigungsorgan (7) kommt zur Halterung der Muschel (**6**) mit dem Tragelement (**2**) in Eingriff. Der Kopfbügel (**1**) besitzt ein sich über den Bereich der Schädeldecke des Benutzers erstreckendes Bogenteil (**3**), welches durchgehende Öffnungen (**30**) zur Belüftung der vom Bogenteil (**3**) überdeckten Partie der Schädeldecke des Benutzers aufweist.

## Beschreibung

### Anwendungsgebiet der Erfindung

Die vorliegende Erfindung bezieht sich auf einen Muschelgehörschützer mit einem elastischen Kopfbügel und beidseits des Kopfbügels einer jeweils angeordneten Muschel. Zur Halterung der Muscheln sind am Kopfbügel Tragelemente und an den Muscheln Befestigungsorgane vorgesehen. Derartige Muschelgehörschützer umkapseln die Ohren des Benutzers und dienen zur Schalldämmung, um die Lärmeinwirkung auf das menschliche Gehör zu verringern. Speziell befasst sich die Erfindung mit der Konstruktion von Kopfbügel, Tragelement und Befestigungsorgan.

### Stand der Technik

Der Katalog "Arbeitsschutz - Sicherheit 1999, 2000, 2001" der ASSI-Arbeitsschutz GmbH & Co. KG, Berlin, enthält z.B. auf Seite 27, oben, einen Muschelgehörschützer der Typenbezeichnung "Peltor H6" mit Kopfbügel. Der gezeigte Kopfbügel weist zwei Haltebügel aus Draht auf, die in ein geschlossenes Bogenteil aus Kunststoff eingeschweisst sind. An den Enden der Haltebügel befinden sich Tragelemente, die Befestigungsnocken an den Muscheln fixiert sind. Zur Höheneinstellung der Muscheln lassen sich die Enden der Haltebügel verschieden tief in die Tragelemente einschieben. Diese Konstruktion hat wesentliche Unvollkommenheiten, nämlich:
- Das relativ breite und geschlossene Kunststoffbogenteil verursacht insbesondere bei körperlicher physischer Anstrengung einen Feuchtigkeitsstau unter dem Bogenteil, da der dort entstehende Körperschweiss nicht abgeführt werden kann.
- Die Haltebügel sind lose verschiebbar im Bogenteil eingebettet, was den ganzen Gehörschützer in der Handhabung instabil macht.
- Die in den Tragelementen steckenden Enden der Haltebügel ergeben mangels stabilerer Führung eine zusätzliche Labilität des Gehörschützers beim Verstellen.
- Schliesslich ist das Aufbringen des Bogenteils und das Einbetten der Haltebügel für eine Serienproduktion relativ arbeitsaufwendig.

### Aufgabe der Erfindung

Angesichts der zuvor geschilderten Nachteile der aus dem Stand der Technik vorbekannten Muschelgehörschützer, liegt der Erfindung die Aufgabe zugrunde, die Trageigenschaften des Kopfbügels zu verbessern, den Verstellmechanismus der Muscheln stabiler zu gestalten sowie eine effizientere Serienproduktion zu ermöglichen.

### Übersicht über die Erfindung

Der Muschelgehörschützer besitzt einen elastischen Kopfbügel, an dessen beiden Enden je eine Muschel fixiert ist. Zur Halterung der Muschel weist der Kopfbügel Tragelemente auf, die mit komplementären Befestigungsorganen an der Muschel in Eingriff kommen. Der Kopfbügel besitzt ein sich über den Bereich der Schädeldecke erstreckendes Bogenteil, welches zur Belüftung der vom Bogenteil überdeckten Partie der Schädeldecke des Benutzers durchgehende Öffnungen aufweist.

Die nachfolgenden Merkmale betreffen vorteilhafte Ausgestaltungen des Muschelgehörschützers. Im Bogenteil ist zumindest ein Haltebügel fest eingebettet, der auf beiden Seitenrändern aus dem Bogenteil herausragt. An beiden Enden des zumindest einen Haltebügels sind die Tragelemente zum Verbinden mit den Befestigungsorganen der beiden Muscheln angeordnet. Im Bogenteil, welches vorteilhaft vorzugsweise im Kunststoffspritzverfahren hergestellt wird, sind zwei Haltebügel, in der Regel aus Draht, fest eingebettet, die auf beiden Seiten aus dem Bogenteil herausragen. An beiden Enden eines jeden Haltebügels sind jeweils ein Tragelement zum Verbinden mit den Befestigungsorganen der beiden Muscheln vorgesehen. Beide Haltebügel sind im Bogenteil nahe dessen Längsränder eingebettet. Hinter den beiden Seitenrändern, wo die Haltebügel aus dem Bogenteil austreten, spreizen sich diese gabelförmig, um mit den an den Enden der Haltebügel angeordneten Tragelementen die Muscheln seitlich an den Befestigungsorganen zu fassen.

Die Öffnungen sind sich längs zwischen den Aussenrändern erstreckende, systematisch gebildete Schlitze, wobei zwischen den Öffnungen Querstege verbleiben, welche die Aussenränder verbinden. Als Öffnungen kämen aber auch sonstige Geometrien vieleckiger oder rundlicher Gestalt in Betracht. Das Tragelement hat die Gestalt einer länglichen Spange, worin ein sich axial erstreckender Führungsschlitz verläuft. Auf der der Muschel zugewandten Innenseite des Tragelements umgibt ein Freischnitt den Führungsschlitz, so dass den Führungsschlitz eine Schulterfläche und eine dazu senkrechte Bande umläuft, die von Seiten der Muschel zugänglich sind. Die Bande hat an ihren Längsseiten ein eine Wellenkontur ergebendes Kerbenraster mit Tälern und Kämmen. Täler und Kämme sind deckungsgleich jeweils auf gleicher Höhe beidseits des Führungsschlitzes positioniert.

Das Befestigungsorgan besitzt die Gestalt eines Nockens, von denen zwei an einer Muschel vorgesehen sind. Die Befestigungsorgane sind symmetrisch, an den beiden Seitenflanken der Muschel positioniert. Ein Befestigungsorgan besteht zunächst aus einem Sockel, der sich von der Seitenflanke der Muschel erhebt und dazu bestimmt ist, im montierten Zustand im Kerbenraster zu sitzen. Ferner weist das- Befestigungsorgan einen sich aus dem Sockel erhebenden Mittelabschnitt auf, der im montierten Zustand im Führungsschlitz sitzt und gegenüber dem Sockel von vermindertem Querschnitt ist, so dass am Übergang vom Mittelabschnitt zum Sockel ein absatzförmiger Anschlag entsteht. Schliesslich hat das Befestigungsorgan zuvorderst einen, dessen freies Ende bildenden Zapfen, welcher breiter als der Führungsschlitz ist und im montierten Zustand an der Aussenseite des Tragelements anliegt.

Der Sockel hat einen kreisförmigen und der Mittelabschnitt einen linsenförmigen Querschnitt, wobei die Scheitelpunkte des letzteren oben und unten liegen. Im montierten Zustand kommt der Sockel zwischen zwei gegenüber liegenden Tälern des Kerbrasters zu liegen.

Bei einer gewünschten Höhenverstellung der Muscheln lässt sich der Sockel innerhalb des Kerbrasters mit Widerstand über die Kämme schieben, wobei der Anschlag des Befestigungsorgans auf der Schulterfläche des Tragelements aufsetzt. Die Muschel ist leichtgängig um eine Achse schwenkbar, welche sich von einem Befestigungsorgan durch die Muschel hindurch zum gegenüber liegenden Befestigungsorgan erstreckt. Der Schwenkbereich - zur Anpassung an die Anatomie des Benutzers - wird durch die Scheitelpunkte begrenzt. Infolge der Elastizität der Tragelemente lassen sich die Zapfen von der Innenseite her durch den in der Breite engeren Führungsschlitz drücken.

Die besonderen Vorteile des erfindungsgemässen Muschelgehörschützers liegen, nach Erfüllung der durch die Aufgabe vorgegebenen Ziele, in der entscheidend verbesserten Benutzerfreundlichkeit sowohl beim Tragen des Gehörschützers als auch beim Einstellen auf die individuelle Kopfanatomie. Die bisher lästige, sich aufstauende Schweissbildung unter dem Bogenteil wird vermieden. Das Gerät als Ganzes ist von soliderer Konstruktion, was sich besonders beim Auf- und Absetzen sowie beim Verstellen bemerkbar macht. Schliesslich erlaubt die Weiterentwicklung eine effizientere Serienproduktion.

### Kurzbeschreibung der beigefügten Zeichnungen

Es zeigen:

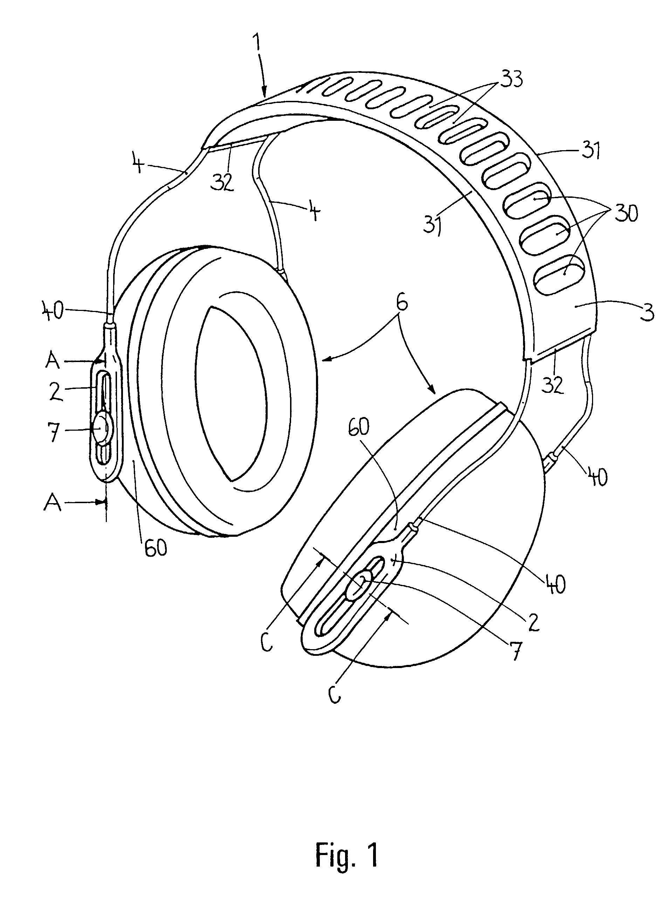

- Figur 1 -: einen kompletten Muschelgehörschützer mit Kopfbügel und eingehängten Muscheln in Perspektivdarstellung;

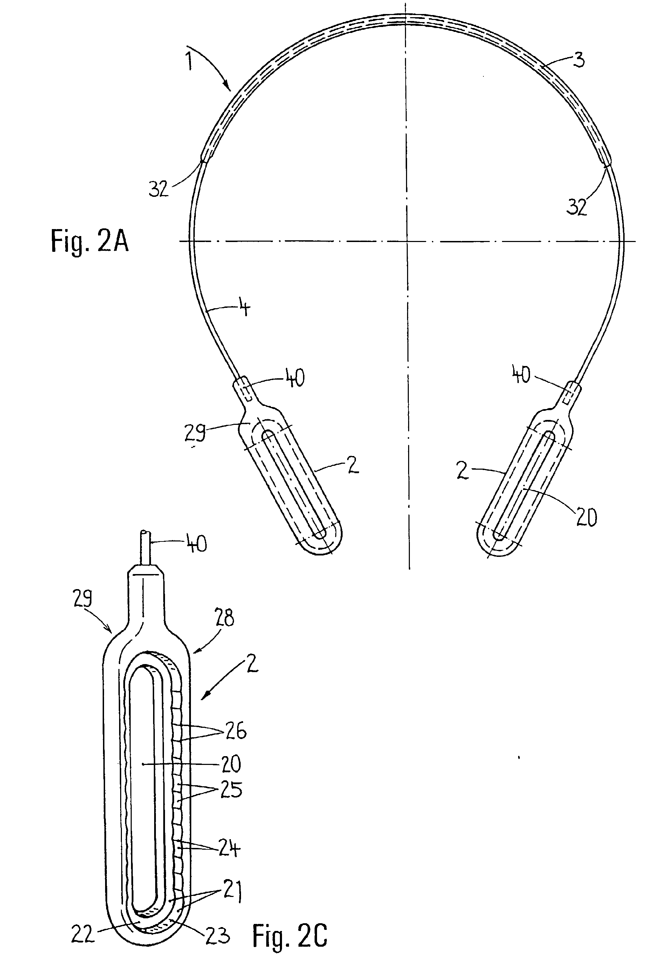

- Figur 2A -: den separaten Kopfbügel in Frontansicht;

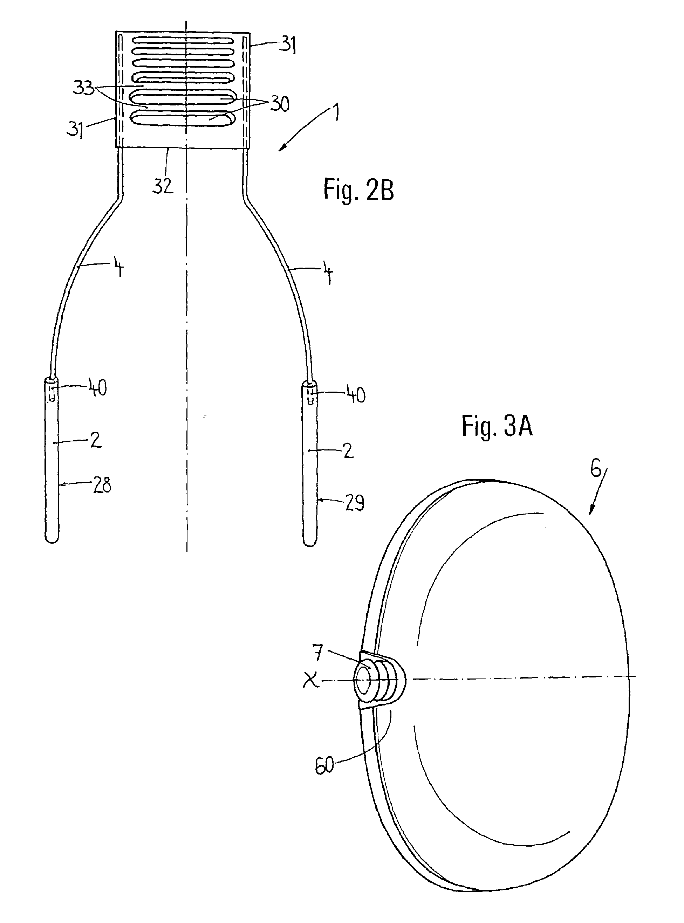

- Figur 2B -: den Kopfbügel gemäss Figur 2A in Seitenansicht;
- Figur 2C -: ein Tragelement des Kopfbügels, mit Blick auf dessen Innenseite, in Perspektivdarstellung;
- Figur 3A -: eine separate Muschel in Perspektivdarstellung;

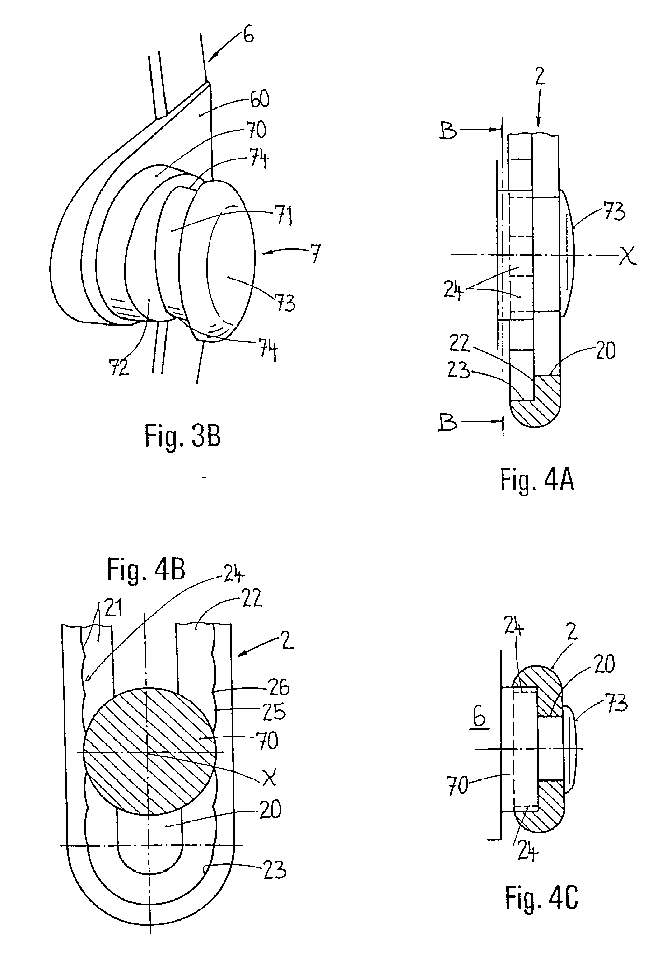

- Figur 3B -: ein Befestigungselement der Muschel gemäss Figur 3A vergrössert in Perspektivdarstellung;
- Figur 4A -: das Befestigungsorgan im Tragelement eingerastet als Teilschnitt aus Figur 1A längs der Linie A-A;
- Figur 4B -: das Befestigungsorgan im Tragelement eingerastet als Schnitt aus Figur 4A längs der Linie B-B; und
- Figur 4C -: das Befestigungsorgan im Tragelement eingerastet als Schnitt aus Figur 1 längs der Linie C-C.

### Ausführungsbeispiel

Mit Bezug auf die beiliegenden Zeichnungen erfolgt nachstehend die detaillierte Beschreibung eines bevorzugten Ausführungsbeispiels zum erfindungsgemässen Muschelgehörschützer.

Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden oder nachfolgenden Figurenbeschreibungen Bezug genommen. Im Interesse der Übersichtlichkeit wird auf die wiederholte Bezeichnung von Bauteilen in weiteren Figuren zumeist verzichtet, sofern zeichnerisch eindeutig erkennbar ist, dass es sich um "wiederkehrende" Bauteile handelt.

### Figur 1

Der Muschelgehörschützer besteht - in der Grobstruktur betrachtet - aus einem elastischen Kopfbügel **1** und zwei am Kopfbügel **1** angeordneten Muscheln **6**. Der Kopfbügel **1** besitzt ein sich über den Bereich der Schädeldecke erstreckendes Bogenteil **3**, welches zur Belüftung der vom Bogenteil **3** überdeckten Partie der Schädeldecke des Benutzers durchgehende Öffnungen **30** aufweist. Die Öffnungen **30** sind z.B. sich längs zwischen den Aussenrändern **31** erstreckende, systematisch angeordnete Schlitze, wobei zwischen den Öffnungen **30** die Aussenränder **31** verbindende Querstege **33** verbleiben. Vieleckige oder rundliche Geometrien wären gleichermassen für die Öffnungen **30** geeignet. Im Bogenteil **3**, welches vorzugsweise im Kunststoffspritzverfahren hergestellt wird, sind nahe den Längsrändern **31** zwei Haltebügel **4**, vorrangig aus Draht, fest eingebettet, die auf beiden Seitenränder **32** aus dem Bogenteil (**3**) herausragen. Hinter den beiden Seitenrändern **32**, wo die Haltebügel **4** aus dem Bogenteil **3** austreten, spreizen sich die beiden Haltebügel **4** gabelförmig, um mit den an den Enden **40** der Haltebügel 4 sitzenden Tragelementen **2** die Muscheln **6** seitlich an Befestigungsorganen **7** zu fassen. Das Tragelement **2** hat die Gestalt einer länglichen Spange und das Befestigungsorgan **7** die eines Nockens. An jeder Muschel **6** sind symmetrisch zwei Befestigungsorgane **7** an den beiden Seitenflanken **60** vorgesehen.

### Figuren 2A bis 2C

Im spangenförmigen Tragelement **2** gibt es einen sich axial erstreckenden Führungsschlitz **20**. Auf der der Muschel **6** zugewandten Innenseite **28** des Tragelements **2** läuft um den Führungsschlitz **20** ein Freischnitt **21** um, wodurch der Führungsschlitz **20** von einer Schulterfläche **22** und einer dazu senkrechten Bande **23** umgeben wird, die von Seiten der Muschel **6** zugänglich sind. Die Bande **23** besitzt an ihren Längsseiten ein eine Wellenkontur ergebendes Kerbenraster **24** mit Tälern **25** und Kämmen, wobei die Täler **25** und Kämme **26** jeweils auf gleicher Höhe beidseits des Führungsschlitzes **20** positioniert sind. Vorteilhaft sind auch die Tragelemente **2** an die Enden **40** der Haltebügel **4** als Kunststoffteile angegossen.

### Figuren 3A bis 4C

Ein Befestigungsorgan **7** besteht zunächst aus einem im Querschnitt kreisrunden Sockel **70**, der sich von der Seitenflanke **60** der Muschel **6** erhebt und dazu bestimmt ist, im montierten Zustand im Kerbenraster **24** zu sitzen. Das Befestigungsorgan **7** weist ferner einen sich aus dem Sockel **70** erhebenden Mittelabschnitt **71** auf, der im montierten Zustand im Führungsschlitz **20** sitzt und einen gegenüber dem Sockel **70** verminderten Querschnitt besitzt, so dass am Übergang vom Mittelabschnitt **71** zum Sockel **70** ein absatzförmiger Anschlag **72** entsteht. Schliesslich hat das Befestigungsorgan **7** zuvorderst, einen das freie Ende bildenden Zapfen **73**, welcher die Breite des Führungsschlitzes **20** überragt und im montierten Zustand an der Aussenseite **29** des Tragelements **2** zu liegen kommt. Der Mittelabschnitt **71** hat einen linsenförmigen Querschnitt, dessen Scheitelpunkte **74** sich oben und unten befinden.

Im montierten Zustand nimmt der Sockel **70** zwischen zwei gegenüber liegenden Tälern **25** des Kerbrasters **24** seine Position ein. Bei einer gewünschten Höhenverstellung der Muscheln **6** lässt sich der Sockel **70** innerhalb des Kerbrasters **24** gegen einen spürbaren Widerstand über die Kämme **26** schieben. Der Anschlag **72** des Befestigungsorgans **7** setzt auf der Schulterfläche **22** des Tragelements **2** auf. Die Muschel **6** schwenkt leichtgängig um eine Achse **X**, welche sich von einem Befestigungsorgan **7** durch die Muschel **6** hindurch zum gegenüber liegenden Befestigungsorgan **7** erstreckt. Der limitierte Schwenkbereich ergibt sich aus der Abrollstrecke auf dem Mittelabschnitt **71** zwischen dessen beiden Scheitelpunkten **74** und dient zur Anpassung an die Anatomie des Benutzers. Aufgrund der Elastizität des Tragelements **2** lässt sich der Zapfen **73** von der Innenseite **28** durch den in der Breite engeren Führungsschlitz **20** drücken.

## Patentansprüche

1. Muschelgehörschützer mit:
a) einem elastischen Kopfbügel (**1**);
b) beidseits am Kopfbügel (**1**) jeweils einer angeordneten Muschel (**6**);
c) zumindest einem Tragelement (**2**) am Kopfbügel (**1**); und
d) zumindest einem komplementärem Befestigungsorgan (**7**) an jeder Muschel (**6**), welches zur Halterung der Muschel (**6**) mit dem Tragelement (**2**) in Eingriff kommt, dadurch gekennzeichnet, dass
e) der Kopfbügel (**1**) ein sich über den Bereich der Schädeldecke erstreckendes Bogenteil (**3**) besitzt, welches zur Belüftung der vom Bogenteil (**3**) überdeckten Partie der Schädeldecke des Benutzers durchgehende Öffnungen (**30**) aufweist.

2. Muschelgehörschützer nach Anspruch 1, dadurch gekennzeichnet, dass
a) im Bogenteil (**3**) zumindest ein Haltebügel (**4**) fest eingebettet ist, der auf beiden Seitenrändern (**32**) aus dem Bogenteil (**3**) herausragt; und
b) an beiden Enden (**40**) des zumindest einen Haltebügels (**4**) die Tragelemente (**2**) zum Verbindung mit den Befestigungsorganen (**7**) der beiden Muscheln (**6**) angeordnet sind.

3. Muschelgehörschützer nach Anspruch 1 oder 2, dadurch gekennzeichnet, dass
a) im Bogenteil (**3**), welches im Kunststoffspritzverfahren hergestellt wird, zwei Haltebügel (**4**), vorzugsweise aus Draht, fest eingebettet sind, die auf beiden Seiten aus dem Bogenteil (**3**) herausragen; und
b) an beiden Enden (**40**) eines jeden Haltebügels (**4**) jeweils ein Tragelement (**2**) zum Verbindung mit den Befestigungsorganen (**7**) der beiden Muscheln (**6**) angeordnet sind.

4. Muschelgehörschützer nach Anspruch 3, dadurch gekennzeichnet, dass die beiden Haltebügel (**4**)
a) nahe den Längsrändern (**31**) im Bogenteil (**3**) eingebettet sind; und
b) hinter den beiden Seitenrändern (**32**), wo die Haltebügel (**4**) aus dem Bogenteil (**3**) austreten, sich gabelförmig spreizen, um mit den an den Enden (**40**) der Haltebügel (**4**) sitzenden Tragelementen (**2**) die Muscheln (**6**) seitlich an den Befestigungsorganen (**7**) zu fassen.

5. Muschelgehörschützer nach Anspruch 1 und 4, dadurch gekennzeichnet, dass die Öffnungen (**30**) vieleckiger, rundlicher oder kombinierter Kontur sind.

6. Muschelgehörschützer nach Anspruch 5, dadurch gekennzeichnet, dass
a) die Öffnungen (**30**) sich längs zwischen den Aussenrändern (**31**) erstreckende, systematisch angeordnete Schlitze sind; und
b) zwischen den Öffnungen (**30**) die Aussenränder (**31**) verbindende Querstege (**33**) verbleiben.

7. Muschelgehörschützer nach Anspruch 1 oder 4, dadurch gekennzeichnet, dass das Tragelement (**2**)
a) die Gestalt einer länglichen Spange hat;
b) darin ein sich axial erstreckender Führungsschlitz (**20**) vorgesehen ist; und
c) auf der der Muschel (**6**) zugewandten Innenseite (**28**) des Tragelements (**2**) um den Führungsschlitz (**20**) ein Freischnitt (**21**) umläuft, wodurch der Führungsschlitz (**20**) von einer Schulterfläche (**22**) und einer dazu senkrechten Bande (**23**) umgeben wird, die von Seiten der Muschel (**6**) zugänglich sind.

8. Muschelgehörschützer nach Anspruch 7, dadurch gekennzeichnet, dass
a) die Bande (**23**) an ihren Längsseiten ein eine Wellenkontur ergebendes Kerbenraster (**24**) mit Tälern (**25**) und Kämmen (**26**) aufweist; wobei
b) die Täler (**25**) und Kämme (**26**) jeweils auf gleicher Höhe beidseits des Führungsschlitzes (**20**) positioniert sind.

9. Muschelgehörschützer nach einem der Ansprüche 1 bis 4, dadurch gekennzeichnet, dass das Befestigungsorgan (**7**)
a) die Gestalt eines Nockens hat;
b) zwei Befestigungsorgane (**7**) an einer Muschel (**6**) vorgesehen sind; und
c) die Befestigungsorgane (**7**) symmetrisch, an den beiden Seitenflanken (**60**) der Muschel (**6**) angeordnet sind.

10. Muschelgehörschützer nach einem der Ansprüche 1 bis 4 und 9, dadurch gekennzeichnet, dass das Befestigungsorgan (**7**) besteht aus:
a) einem Sockel (**70**), der sich von der Seitenflanke (**60**) der Muschel (**6**) erhebt und dazu bestimmt ist, im montierten Zustand im Kerbenraster (**24**) zu sitzen;
b) einem sich aus dem Sockel (**70**) erhebenden Mittelabschnitt (**71**), der im montierten Zustand im Führungsschlitz (**20**) sitzt und einen gegenüber dem Sockel (**70**) verminderten Querschnitt aufweist, wodurch am Übergang vom Mittelabschnitt (**71**) zum Sockel (**70**) ein absatzförmiger Anschlag (**72**) entsteht; und
c) einem zuvorderst, das freie Ende des Befestigungsorgans (**7**) bildenden Zapfen (**73**), welcher die Breite des Führungsschlitzes (**20**) überragt und im montierten Zustand an der Aussenseite (**29**) des Tragelements (**2**) zu liegen kommt.

11. Muschelgehörschützer nach Anspruch 10, dadurch gekennzeichnet, dass
a) der Sockel (**70**) einen kreisförmigen Querschnitt hat;
b) der Mittelabschnitt (**71**) einen linsenförmigen Querschnitt hat, dessen Scheitelpunkte (**74**) oben und unten liegen; und im montierten Zustand:
c) der Sockel (**70**) zwischen zwei gegenüber liegenden Tälern (**25**) des Kerbrasters (**24**) zu liegen kommt;
d) bei einer gewünschten Höhenverstellung der Muscheln (**6**) der Sockel (**70**) innerhalb des Kerbrasters (**24**) mit Widerstand über die Kämme (**26**) schiebbar ist;
e) der Anschlag (**72**) des Befestigungsorgans (**7**) auf der Schulterfläche (**22**) des Tragelements (**2**) aufsetzt;
f) die Muschel (**6**) leichtgängig um eine Achse (**X**), welche sich von einem Befestigungsorgan (**7**) durch die Muschel (**6**) hindurch zum gegenüber liegenden Befestigungsorgan (**7**) erstreckt, zwischen den Scheitelpunkten (**74**) begrenzt zur Anpassung an die Anatomie des Benutzers schwenkbar ist; und
g) durch Elastizität des Tragelements (**2**) sich der Zapfen (**73**) von der Innenseite (**28**) durch den in der Breite engeren Führungsschlitz (**20**) drücken lässt.
